# EUROPÄISCHE PATENTSCHRIFT

(11) **EP 0 987 249 B1**
(45) Veröffentlichungstag und Bekanntmachung des Hinweises auf die Patenterteilung: **17.03.2004**
(21) Anmeldenummer: 99115060.8
(22) Anmeldetag: 04.08.1999
(51) Int. Cl.: C07C 281/02

(54) **Verfahren zur Herstellung von Benzylcarbazaten**
Process for the preparation of benzyl carbazates
Procédé pour la préparation de carbazates benzyliques

(30) Priorität: 17.08.1998 DE 19837070
(43) Veröffentlichungstag der Anmeldung: 22.03.2000
(73) Patentinhaber: Bayer Chemicals AG, 51368 Leverkusen (DE)
(72) Erfinder: Ritzer, Edwin, Dr., 51381 Leverkusen (DE); Söllner, Robert, Dr., 51373 Leverkusen (DE); Dreisbach, Claus, Dr., 51065 Köln (DE); Jelitto, Frank, Dr., 51467 Bergisch Gladbach (DE)

(56) Entgegenhaltungen:
- EP-A- 0 770 598
- M. SELVA ET AL.: "Selective mono-benzylation of methylene active compounds with dibenzyl carbonate: benzylation of phenol" JOURNAL CHEM. SOC. PERKIN TRANS. 1, Bd. 15, 1995, Seiten 1889-1894, XP002124351
- RABJOHN ET AL.: "The synthesis and Reactions of Disazodicarboxylates *" JOURNAL AMERICAN CHEMICAL SOCIETY, Bd. 70, 1948, Seite 1181-1183 XP000617257

## Beschreibung

Die vorliegende Erfindung betrifft ein Verfahren zur Herstellung von gegebenenfalls substituierten Benzylcarbazaten durch Umsetzung von C₁-C₈-Dialkylcarbonaten mit gegebenenfalls substituierten Benzylalkoholen in Gegenwart eines Katalysators und anschließende Umsetzung mit Hydrazinhydrat.

Carbazate werden in zunehmendem Ausmaß als Zwischenprodukte zur Herstellung von Pflanzenschutzmitteln und Pharmazeutika eingesetzt. Insbesondere werden sie zur Synthese von Peptiden verwendet (EP-A 0 106 282). Nach J. Biol. Chem. 266, 5525 (1991) läßt sich aus Benzylcarbazat ferner Hydrazinosuccinat, ein Inhibitor der Aspartat-aminotransferase, herstellen. Die EP-A 0 143 078 beschreibt den Einsatz von Benzylcarbazat zur Herstellung von Pflanzenschutzmitteln. Der Herstellung von Carbazaten kommt vor diesem Hintergrund eine erhöhte Bedeutung zu.

Carbazate werden im allgemeinen durch Umsetzung von Chlorameisensäureestern mit Hydrazin gewonnen. So wird in Chem. Ber. 92, 1478 (1959) die Reaktion von Chlorameisensäurebenzylester mit Hydrazin unter Bildung von Benzylcarbazat beschrieben. Hierbei ist jedoch die Herstellung der Vorprodukte, die unter Verwendung von Phosgen erfolgt, vergleichsweise aufwendig, und die Reinheit der erhaltenen Carbazate ist ferner für manche Einsatzzwecke nicht ausreichend, da im Verlauf der mehrstufigen Synthese aus thermisch empfindlichen Zwischenprodukten Nebenprodukte entstehen, die das gewünschte Carbazat verunreinigen.

In J. Chem. Soc. Perkin Trans. 1, Bd. 15, 1995, 1889-1894 wird die Herstellung von Dibenzylcarbonat durch Umsetzung von Benzylalkohol mit Dimethylcarbonat in Gegenwart von Kaliumcarbonat als Katalysator beschrieben. Hierbei werden 51,7 Gew.-% Kaliumcarbonat bezogen auf das eingesetzte Dimethylcarbonat verwendet und eine Ausbeute von 19 % bezogen auf Dimethylcarbonat erhalten.

Nachteilig an diesem Verfahren ist die große Menge an Katalysator bei gleichzeitig nicht zufriedenstellenden Umsätzen.

Nach einer anderen Methode werden symmetrische Dialkylcarbonate mit Hydrazin umgesetzt (J. Am. Chem. Soc. 70, 1181 (1948)). Auch hier ist die Herstellung der Vorprodukte schwierig oder unmöglich, vor allem, wenn es sich um höhere Carbonate handelt. Außerdem ist diese Verfahrensweise problematisch, da bei der Isolierung des Produkts als Hydrochlorid die Bildung von chlorierten, z.T. korrosiven Verbindungen im Zuge der Aufarbeitung nicht vollständig vermieden werden kann.

Aus EP-A 0 770 598 ist ferner ein Verfahren zur Herstellung von gegebenenfalls substituierten C₂-C₂₀-Alkyl-, C₃-C₆-Cycloalkyl- und C₆-C₁₂-Arylcarbazaten bekannt, bei dem man ein unsubstituiertes C₁-C₄-Alkylcarbazat mit einem gegebenenfalls substituierten C₂-C₂₀-Alkyl-, C₃-C₆-Cycloalkyl- oder C₆-C₁₂-Arylalkohol in Gegenwart eines Katalysators umsetzt. Das unsubstituierte C₁-C₄-Alkylcarbazat ist dabei durch Umsetzung von Dialkylcarbonaten mit Hydrazin zugänglich (siehe z.B. Ber. Chem. Ges. 47, 2183-2188). Während die Reinheit der erhaltenen Alkyl-, Cycloalkyl- oder Arylcarbazate gut ist und beispielsweise bei 97 % liegt, ist die Ausbeute des Verfahrens an dem Carbazat bezogen auf das eingesetzte Alkylcarbazat relativ gering und beträgt beispielsweise nur ca. 65 %.

Aufgrund des steigenden Bedarfs an Carbazaten, insbesondere an Benzylcarbazaten, bestand daher die Aufgabe, ein technisch einfach durchzuführendes Verfahren zur Herstellung von Benzylcarbazaten in hoher Reinheit bereitzustellen.

Gelöst wird diese Aufgabe durch ein Verfahren zur Herstellung von gegebenenfalls substituierten Benzylcarbazaten der Formel (I) 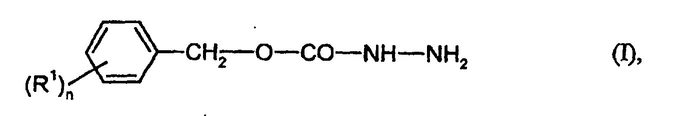 worin
- R¹: unabhängig voneinander C₁-C₄-Alkyl, C₁-C₄-Alkoxy, Di-C₁-C₄-Alkylamino, Nitro, Halogen, Hydroxy, die Gruppe X-R³, worin X für O oder S und R³ für geradkettiges oder verzweigtes C₁-C₄-Alkyl steht, die Gruppe COOR⁴, wobei R⁴ für C₁-C₄-Alkyl steht, die Gruppe NR⁵R⁶, wobei R⁵ und R⁶ unabhängig voneinander jeweils für C₁-C₄-Alkyl stehen, oder eine gegebenenfalls mit geradkettigen oder verzweigten C₁-C₄-Alkylgruppen substituierte C₃-C₆-Cycloalkylgruppe darstellt und
- n: eine ganze Zahl von 0 bis 5 ist,
wobei man in einer ersten Stufe ein Dialkylcarbonat der Formel (II)

R²-O-CO-O-R² (II),

worin
- R²: für einen verzweigten oder unverzweigten C₁-C₈-Alkylrest steht,
mit einem Benzylalkohol der Formel (III) 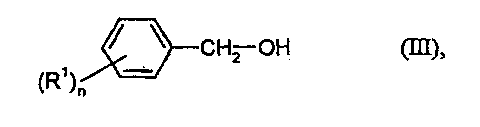 worin R¹ und n die für die Formel (I) angegebenen Bedeutungen besitzen,
in Gegenwart eines Katalysators umsetzt, wobei der Katalysator in einer Menge von 0,1 bis 10 Gew.-%, bezogen auf das Dialkylcarbonat der Formel (II) eingesetzt wird, das erhaltene Reaktionsgemisch vom Katalysator abtrennt und anschließend in der zweiten Stufe mit Hydrazinhydrat umsetzt.

Bei dem in der ersten Stufe des Verfahrens eingesetzten Dialkylcarbonat der Formel (II) steht R² für einen verzweigten oder unverzweigten C₁-C₈-, bevorzugt C₁-C₄-Alkylrest, insbesondere für Methyl oder Ethyl.

Solche Dialkylcarbonate sind gut zugänglich. Dimethylcarbonat ist beispielsweise im großtechnischem Maßstab ohne Einsatz von Phosgen durch Oxidation von Kohlenmonoxid mit Sauerstoff in Gegenwart von Methanol und Katalysatoren herstellbar (EP-A 0 365 083).

In den Benzylalkoholen der Formel (III) ist der Benzylring gegebenenfalls durch einen oder mehrere Reste R¹ substituiert, wobei R¹ gleich oder verschieden ist, und C₁-C₄-, bevorzugt C₁-C₃-Alkyl, C₁-C₄-, bevorzugt C₁-C₃-Alkoxy, Di-C₁-C₄-, bevorzugt Di-C₁-C₃-Alkylamino, Nitro, Halogen, Hydroxy, die Gruppe X-R³, wobei X für Ö oder S und R³ für geradkettiges oder verzweigtes C₁-C₄-Alkyl steht, die Gruppe COOR⁴, wobei R⁴ für C₁-C₄-Alkyl steht, die Gruppe NR⁵R⁶, wobei R⁵ und R⁶ unabhängig voneinander jeweils für C₁-C₄-Alkyl stehen, oder eine gegebenenfalls mit geradkettigen oder verzweigten C₁-C₄-Alkylgruppen substituierte C₃-C₆-Cycloalkylgruppe darstellt n ist eine ganze Zahl von 0 bis 5, bevorzugt 0 bis 3, insbesondere 0 oder 1.

Als Katalysatoren können in der ersten Stufe des erfindungsgemäßen Verfahrens die vielfältigsten basisch reagierenden Verbindungen eingesetzt werden. Aus Gründen der leichteren Abtrennbarkeit nach Beendigung des erfindungsgemäßen Verfahrens sind feste basische Verbindungen als Katalysator bevorzugt. Beispiele für geeignete Katalysatoren sind: Alkalimetallhydroxide wie Lithiumhydroxid, Natriumhydroxid und Kaliumhydroxid, Erdalkalimetallcarbonate und -hydrogencarbonate wie Lithiumcarbonat, Natriumcarbonat, Kaliumcarbonat, Magnesiumcarbonat, Calciumcarbonat, Natriumhydrogencarbonat und Kaliumhydrogencarbonat und Alkalimetall- und Erdalkalimetallalkoholate wie Lithiummethanolat und Natriummethanolat. Bevorzugt werden Alkalimetallcarbonate, insbesondere Natriumcarbonat eingesetzt. Bei Verwendung von Alkalimetall- und Erdalkalimetallalkoholaten wählt man zur Vermeidung der Bildung unerwünschter Nebenprodukte bevorzugt solche aus, die sich vom jeweils eingesetzten Alkohol der Formel (III) ableiten.

Der Katalysator wird erfindungsgemäß in einer Menge von 0,1 bis 10 Gew.-%, bevorzugt 0,5 bis 5 Gew.-%, und insbesondere 1 bis 3 Gew.-%, bezogen auf das Dialkylcarbonat der Formel (II), eingesetzt.

Gegebenenfalls wird die Umsetzung der Dialkylcarbonate der Formel (II) mit den Benzylalkoholen der Formel (III) in der ersten Stufe in Gegenwart eines organischen Lösungsmittels durchgeführt, das unter den gewählten Reaktionsbedingungen stabil ist und sich gegenüber den eingesetzten Dialkylcarbonaten und Benzylalkoholen sowie den erhaltenen Dibenzylcarbonaten inert verhält.

Hierbei kann es sich um Kohlenwasserstoffe, Ether und Säureamide handeln, wie Cyclohexan, Toluol, Chlorbenzol, Ethylen- und Diethylenglykoldimethylether sowie Dimethylformamid.

Zur Durchführung der ersten Stufe des erfindungsgemäßen Verfahrens werden das jeweilige Dialkylcarbonat der Formel (II) und der jeweilige Benzylalkohol der Formel (III) in einem Mol-Verhältnis von (1-8):1, bevorzugt (1,5-6):1 und insbesondere (2-4):1 eingesetzt.

Die erste Stufe des Verfahrens wird üblicherweise bei Temperaturen von 60 bis 160°C, bevorzugt 80 bis 140°C, und einem Druck von 0,0001 bis 0,1 MPa (0,001 - 1 bar) durchgeführt.

Der im Verlauf der ersten Stufe gebildete Alkohol R²-OH wird bevorzugt bereits während der Umsetzung abdestilliert. Dieser Vorgang kann dadurch gefördert werden, daß das in der ersten Stufe eingesetzte Lösungsmittel, etwa ein aromatischer Kohlenwasserstoff, insbesondere Toluol, mit dem Alkohol R²-OH ein Azeotrop bildet.

Gegebenenfalls kann der Druck gegen Ende der Umsetzung in der ersten Stufe auch auf unter 0,0001 MPa (1 mbar) abgesenkt werden.

Nach Beendigung der ersten Stufe wird das Reaktionsgemisch aufgearbeitet, indem man den Katalysator abtrennt, beispielsweise durch Sedimentation, Filtration öder Extraktion mit Wasser. Eventuell noch vorhandener überschüssiger Benzylalkohol oder überschüssiges Dialkylcarbonat kann gegebenenfalls durch Destillation abgetrennt werden. Das verbleibende rohe Reaktionsprodukt kann unmittelbar in die Umsetzung der zweiten Stufe eingesetzt werden, oder aber zuvor noch durch einen zusätzlichen Destillationsschritt gereinigt werden.

In der zweiten Stufe des erfindungsgemäßen Verfahrens wird das Reaktionsgemisch der ersten Stufe mit Hydrazinhydrat umgesetzt. Es hat sich bewährt, bei Temperaturen von 60 bis 160°C, bevorzugt 80 bis 140°C und einem Druck von 0,01 bis 1 MPa (0,1 - 10 bar), vorzugsweise 0,1 MPa (1 bar) zu arbeiten. Der Zusatz eines Katalysators ist in dieser zweiten Stufe nicht erforderlich.

Das Hydrazinhydrat wird in einem Mol-Verhältnis von (1-3):1, bevorzugt (1-2):1, bezogen auf das Dibenzylcarbonat eingesetzt. Der im Verlauf der zweiten Stufe gebildete Alkohol R²OH wird analog zur ersten Stufe bereits während der Umsetzung abdestilliert, insbesondere in Form eines Azeotrops mit einem aromatischen Kohlenwasserstoff, wie Toluol, der als Lösungsmittel zugegen ist. Zusätzlich oder alternativ kann der Druck gegen Ende der Umsetzung in der zweiten Stufe auf unter 10⁻⁴ MPa (1 mbar) abgesenkt werden.

Nach Beendigung der Umsetzung in der zweiten Stufe kann das Reaktionsgemisch mit bekannten Techniken aufgearbeitet werden, z.B. durch Destillation, insbesondere durch Wasserdampfdestillation, oder durch Kristallisation, insbesondere durch Lösungs- oder Schmelzkristallisation.

Das erfindungsgemäße Verfahren ermöglicht die Bereitstellung der gewünschten, gegebenenfalls substituierten Benzylcarbazate auf technisch einfache Weise. Es müssen keine schwierig zu handhabenden Chemikalien eingesetzt werden, und es entstehen auch keine aufwendig zu entsorgenden Abfälle. Das Verfahren zeichnet sich ferner dadurch aus, daß die gewünschten Benzylcarbazate in hoher Reinheit erhalten werden, d.h. frei von nicht oder nur schwer abtrennbaren Nebeükomponenten, die in Folgeumsetzungen stören. Wesentlich ist insbesondere, daß chloridfreie und damit nicht korrosiv wirkende Benzylcarbazat-Produkte erhalten werden. Eine weitere Reinigung der Benzylcarbazate vor dem Einsatz in Folgereaktionen ist daher von Ausnahmefällen abgesehen nicht notwendig.

Von Vorteil sind außerdem die schonenden Reaktionsbedingungen des Verfahrens, durch die auch die Herstellung selbst thermisch empfindlicher Benzylcarbazate möglich ist.

### Beispiel 1

### Herstellung von Benzylcarbazat

- 1. Stufe:: Dibenzylcarbonat aus Diethylcarbonat
472 g (4,0 mol) Diethylcarbonat, 864 g (8,0 mol) Benzylalkohol und 8 g Natriumcarbonat werden bei Raumtemperatur gemischt. Unter Rühren wird die Reaktionsmischung zum Sieden erhitzt; das gebildete Ethanol wird zur Vervollständigung der Reaktion abdestilliert. Hierbei steigt die Sumpftemperatur auf 140°C an. Destilliert kein Alkohol mehr ab, so verringert man den Druck unter Konstanthaltung der Sumpftemperatur.
Nach Erreichen eines Drucks von 10⁻⁴ MPa (1 mbar) bei 140°C werden diese Bedingungen noch 1 Stunde beibehalten. Anschließend wird abgekühlt und der Katalysator durch Sedimentation und Filtration abgetrennt. Das nach Abtrennung des Katalysators verbleibende Reaktionsgemisch wird anschließend destillativ gereinigt. Man erhält 855 g Dibenzylcarbonat, 24,4 g Diethylcarbonat 260 g Ethanol, 183,1 g Benzylalkohol, 14,8 g Benzylethylcarbonat und 0,17 g Dibenzylether.
- 2. Stufe:: Herstellung von Benzylcarbazat aus Dibenzylcarbonat
- A:: 2556 g (10,6 mol) Dibenzylcarbonat und 575 g (17,8 mol) Hydrazinhydrat werden bei Raumtemperatur gemischt. Unter Rühren wird die Reaktionsmischung zum Sieden erhitzt; der gebildete Benzylalkohol wird zusammen mit dem Wasser zur Vervollständigung der Reaktion abdestilliert. Hierbei steigt die Sumpftemperatur auf 140°C an. Destilliert kein Alkohol mehr ab, so verringert man den Druck unter Konstanthaltung der Sumpftemperatur.
Nach Erreichen eines Drucks von 10⁻⁴ MPa (1 mbar) bei 140°C werden diese Bedingungen noch 1 Stunde beibehalten. Anschließend wird abgekühlt und das Produkt destillativ aufgearbeitet.

Der Rückstand der Destillation von 1600 g weist folgende Zusammensetzung auf:

| | |
|---|---|
| 97,2 % Benzylcarbazat | (Ausbeute von 88,2 % bezogen auf eingesetztes Dibenzylcarbonat) |
| 0,37 % Benzylalkohol | |
| 0,16 % Dibenzylether | |
| 0,32 % Dibenzylcarbonat | |

Die 2. Stufe wird in weiteren Versuchen B, C und D analog unter Verwendung der in Tabelle 1 angegebenen Mengenverhältnisse von Dibenzylcarbonat und Hydrazinhydrat sowie Reaktionstemperatur durchgeführt. Die erhaltene Menge Benzylcarbazat sowie dessen Reinheit sind ebenfalls in Tabelle 1 zusammengefaßt.

**Tabelle 1**

| Versuch | Dibenzylcarbonat (g) | Hydrazinhydrat (g) | Benzylcarbazat (g) | Reinheit (%) | Temperatur (°C) |
|---|---|---|---|---|---|
| B | 126,5 | 27,5 | 83 | 92,3 | 160 |
| C | 563,3 | 165 | 391 | 73,8 | 165 |
| D | 512,7 | 120 | 386 | 82,6 | 123 |

Dieser Destillationsrückstand läßt sich nach folgenden Methoden weiter aufarbeiten.

### I. Reinigung durch Umkristallisation aus Diethylcarbonat

100 g Benzylcarbazat aus A (97 %ig) und 50 g Diethylcarbonat werden bei Raumtemperatur gemischt und auf 70°C erwärmt. Anschließend wird abgekühlt und angeimpft. Nach der Kristallisation, Abtrennung und Trocknung unter vermindertem Druck bei 40°C erhält man 52 g Benzylcarbazat mit einer Reinheit größer 99,5 %.

### II. Reinigung durch Schmelzen in Gegenwart von Wasser

100 g Benzylcarbazat aus A (97 %ig) und 100 g Wasser werden bei Raumtemperatur gemischt und auf 90°C erwärmt. Anschließend wird auf 40°C abgekühlt. Nach dem Erstarren und Trocknen unter vermindertem Druck bei 40°C erhält man 94 g Benzylcarbonat mit einer Reinheit größer 99,5%.

### III. Reinigung durch Schmelzkristallisation

Ein 2 Zoll-Glasrohr mit Temperiermantel wird unten mit einem Stopfen verschlossen und auf 80°C erwärmt. Dann wird das Rohr mit aufgeschmolzenem, rohem Benzylcarbazat befüllt. Das Rohr wird mit einem zweiten Stopfen, durch den ein Pt100 Thermoelement geführt ist, verschlossen. Dann wird innerhalb von 30 min bis zum Erstarrungspunkt der Einsatzware abgekühlt und die Erstarrungswärme vollständig abgeführt. Dann wird innerhalb von 60 min auf 10°C unter den Erstarrungspunkt abgekühlt und gewartet bis T (innen) und T (Heizmantel) ausgeglichen sind. Durch Entfernen des unteren Stopfens wird der noch flüssige Teil des Produktgemisches (Ablauge) abgetrennt. Die Ablauge enthält Kohlensäuredihydrazid, welches nach Wiederaufschmelzen nicht mehr schmilzt und durch Filtration abgetrennt werden kann.

Danach wird durch langsames Erwärmen des Rohres eine weitere Fraktion (Schwitzfraktion) aufgeschmolzen und abgetrennt Diese Zwischenlauge entspricht in etwa der Einsatzware und kann in die nächste Reinfraktion aufgeschmolzen und als Schmelze gewonnen werden. Durch entsprechende Temperaturprogramme kann die zweite Reinkristallisation bzw. die Ablaugenkristallisation durchgeführt werden. Man erhält 83 g Benzylcarbazat mit einer Reinheit größer 99,0 %.

### IV. Reinigung durch Wasserdampfdestillation

100 g Benzylcarbazat aus A (97 %ig) und 20 g Wasser werden bei Raumtemperatur gemischt und auf 70°C erwärmt. Anschließend wird unter Vakuum (20 mbar) das Wasser zusammen mit den flüchtigen organischen Substanzen abdestilliert; der Sumpf kristallisiert anschließend aus. Man erhält 96 g Benzylcarbazat mit einer Reinheit größer 99,0%.

## Patentansprüche

1. Verfahren zur Herstellung von gegebenenfalls substituierten Benzylcarbazaten der Formel (I) 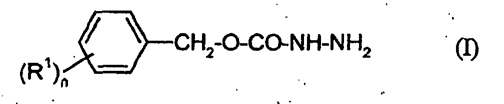 worin
R¹ unabhängig voneinander C₁-C₄-Alkyl, C₁-C₄-Alkoxy, Di-C₁-C₄-Alkylamino, Nitro, Halogen, Hydroxy, die Gruppe X-R³, wobei X für O oder S und R³ für geradkettiges oder verzweigtes C₁-C₄-Alkyl stehen, die Gruppe COOR⁴, wobei R⁴ für C₁-C₄-Alkyl steht, die Gruppe NR⁵ R⁶, wobei R⁵ und R⁶ unabhängig voneinander jeweils für C₁-C₄-Alkyl stehen, oder eine gegebenenfalls mit geradkettigen oder verzweigten C₁-C₄-Alkylgruppen substituierte C₃-C₆-Cycloalkylgruppe darstellt und
n eine ganze Zahl von 0 bis 5 ist,
wobei man in einer ersten Stufe ein Dialkylcarbonat der Formel (II)
R²-O-CO-O-R² (II)
worin
R² für einen verzweigten oder unverzweigten C₁-C₈-Alkylrest steht,
mit einem Benzylalkohol der Formel (III) 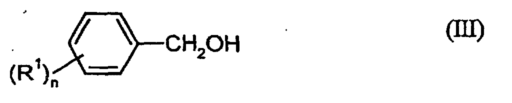 worin
R¹ und n die für die Formel (I) angegebenen Bedeutungen besitzen,
in Gegenwart eines Katalysators umsetzt, wobei der Katalysator in einer Menge von 0,1 bis 10 Gew.-%, bezogen auf das Dialkylcarbonat der Formel (II) eingesetzt wird, das erhaltene Reaktionsgemisch vom Katalysator abtrennt und anschließend mit Hydrazinhydrat umsetzt.

2. Verfahren nach Anspruch 1, wobei in den Dialkylcarbonaten der Formel (II)
R² für einen verzweigten oder unverzweigten C₁-C₄-Alkylrest, bevorzugt für Methyl oder Ethyl steht.

3. Verfahren nach Anspruch 1 oder 2, wobei in den Benzylalkoholen der Formel (III)
R¹ C₁-C₃-Alkyl, C₁-C₃-Alkoxy, Di-C₁-C₃-Alkylamino oder Chlor darstellt, und
n unabhängig davon eine ganze Zahl von 0 bis 3 ist.

4. Verfahren nach einem oder mehreren der Ansprüche 1 bis 3, wobei in der ersten Stufe des Verfahrens ein Alkalimetallcarbonat, insbesondere Natriumcarbonat, als Katalysator eingesetzt wird.

5. Verfahren nach einem oder mehreren der Ansprüche 1 bis 3, wobei der Katalysator in einer Menge von 0,5 bis 5, insbesondere 1 bis 3 Gew.-%, bezogen auf das Dialkylcarbonat der Formel (II), eingesetzt wird.

6. Verfahren nach einem oder mehreren der Ansprüche 1 bis 5, wobei das Verfahren in der ersten Stufe bei einer Temperatur von 60 bis 160°C, bevorzugt 80 bis 140°C und einem Druck von 0,0001 bis 0,1 MPa durchgeführt wird.

7. Verfahren nach einem oder mehreren der Ansprüche 1 bis 6, wobei in der ersten Stufe des Verfahrens das Dialkylcarbonat der Formel (II) mit dem Benzylalkohol der Formel (III) im Molverhältnis von (1-8):1, bevorzugt (1,5-6):1 und insbesondere (2-4):1 eingesetzt werden.

8. Verfahren nach einem oder mehreren der Ansprüche 1 bis 7, wobei das Verfahren in der zweiten Stufe bei einer Temperatur von 60 bis 160°C, bevorzugt 80 bis 140°C und einem Druck von 0,01 bis 1 MPa, bevorzugt 0,1 MPa, durchgeführt wird.

9. Verfahren nach einem oder mehreren der Ansprüche 1 bis 8, wobei in der zweiten Stufe das Hydrazinhydrat, bezogen auf das Dibenzylcarbonat, in einem Molverhältnis von (1-3):1, bevorzugt (1-2):1 eingesetzt wird.

10. Verfahren nach einem oder mehreren der Ansprüche 1 bis 9, wobei das Reaktionsgemisch nach Beendigung der zweiten Stufe durch Destillation, insbesondere Wasserdampfdestillation, oder Kristallisation, insbesondere Lösungs- oder Schmelzkristallisation, aufgearbeitet wird.

## Claims

1. Process for preparing optionally substituted benzyl carbazates of the formula (I) 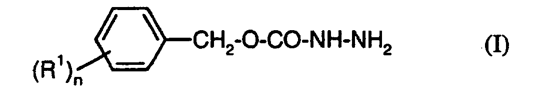 in which
R¹ independently of one another represents C₁-C₄-alkyl, C₁-C₄-alkoxy, di-C₁-C₄-alkylamino, nitro, halogen, hydroxyl, the group X-R³, where X represents O or S and R³ represents straight-chain or branched C₁-C₄-alkyl, the group COOR⁴, where R⁴ represents C₁-C₄-alkyl, the group NR⁵R⁶, where R⁵ and R⁶ independently of one another each represents C₁-C₄-alkyl, or a C₃-C₆-cycloalkyl group which is optionally substituted by straight-chain or branched C₁-C₄-alkyl groups and
n is an integer from 0 to 5,
where in a first step a dialkyl carbonate of the formula (II)
R²-O-CO-O-R² (II)
in which
R² represents a branched or unbranched C₁-C₈-alkyl radical
is reacted with a benzyl alcohol of the formula (III) 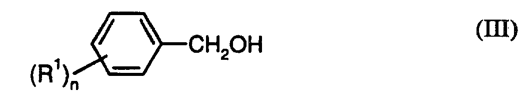 in which
R¹ and n are as defined for the formula (I)
in the presence of a catalyst, where the catalyst is employed in an amount of from 0.1 to 10% by weight, based on the dialkyl carbonate of the formula (II), the resulting reaction mixture is separated off from the catalyst and subsequently reacted with hydrazine hydrate.

2. Process according to Claim 1 where in the dialkyl carbonates of the formula (II)
R² represents a branched or unbranched C₁-C₄-alkyl radical, preferably methyl or ethyl.

3. Process according to Claim 1 or 2 where in the benzyl alcohols of the formula (III)
R¹ represents C₁-C₃-alkyl, C₁-C₃-alkoxy, di-C₁-C₃-alkylamino or chlorine, and
n independently therefrom is an integer from 0 to 3.

4. Process according to one or more of Claims 1 to 3 where the catalyst used in the first step of the process is an alkali metal carbonate, in particular sodium carbonate.

5. Process according to one or more of Claims 1 to 3 where the catalyst is employed in an amount of from 0.5 to 5, in particular from 1 to 3, % by weight, based on the dialkyl carbonate of the formula (II).

6. Process according to one or more of Claims 1 to 5 where the process is carried out in the first step at a temperature of from 60 to 160°C, preferably from 80 to 140°C, and at a pressure of from 0.0001 to 0.1 MPa.

7. Process according to one or more of Claims 1 to 6 where in the first step of the process the dialkyl carbonate of the formula (II) and the benzyl alcohol of the formula (III) are employed in a molar ratio of (1-8):1, preferably (1.5-6):1 and in particular (2-4):1.

8. Process according to one or more of Claims 1 to 7 where the process in the second step is carried out at a temperature of from 60 to 160°C, preferably from 80 to 140°C, and a pressure of from 0.01 to 1 MPa, preferably 0.1 MPa.

9. Process according to one or more of Claims 1 to 8 where in the second step the hydrazine hydrate is employed in a molar ratio of (1-3):1, preferably (1-2):1, based on the dibenzyl carbonate.

10. Process according to one or more of Claims 1 to 9 where the reaction mixture is worked up by distillation, in particular steam distillation, or crystallization, in particular solution or melt crystallization, after the second step has ended.

## Revendications

1. Procédé pour la préparation de benzylcarbazates éventuellement substitués de formule (I) 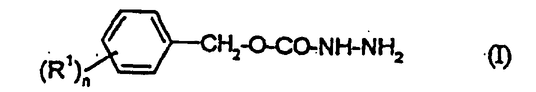 dans laquelle
R¹ désigne, indépendamment l'un de l'autre, un radical alkyle C₁-C₄, alcoxy C₁-C₄, di-C₁-C₄-alkylamino, nitro, un halogène, un radical hydroxy, le groupe X-R³ dans lequel X désigne O ou S et R³ désigne un radical alkyle C₁-C₄ à chaîne droite ou ramifiée, le radical COOR⁴, R⁴ désignant un radical alkyle C₁-C₄, le radical NR⁵R⁶, R⁵ et R⁶ désignant respectivement, indépendamment l'un de l'autre, un radical alkyle C₁-C₄ ou un radical cycloalkyle C₃-C₆ éventuellement substitué par des radicaux alkyle C₁-C₄ à chaîne droite ou ramifiée et
n un nombre entier de 0 à 5,
par lequel, dans une première étape, un dialkylcarbonate de formule (II)
R²-O-CO-O-R² (II)
dans laquelle
R² désigne un radical alkyle C₁-C₈ ramifié ou non ramifié, est mis en réaction avec un alcool benzylique de formule (III) 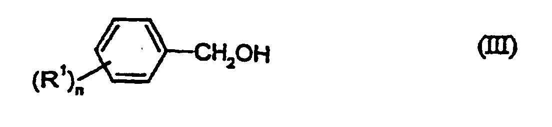 dans laquelle R¹ et n possèdent les significations indiquées pour la formule (I),
en présence d'un catalyseur, le catalyseur étant utilisé dans une quantité de 0,1 à 10% en poids par rapport au carbonate dialkylique de formule (II), le mélange réactif obtenu est séparé du catalyseur et ensuite mis en réaction avec l'hydrate d'hydrazine.

2. Procédé selon la revendication 1,
R² désignant un radical alkyle C₁-C₄ ramifié ou non ramifié, en particulier un radical méthyle ou éthyle
dans les carbonates dialkyliques de formule (II).

3. Procédé selon l'une des revendications 1 ou 2, dans lequel
R¹ représente un radical alkyle C₁-C₃, alcoxy C₁-C₃, di-C₁-C₃-alkylamino ou du chlore et
n représente, indépendamment de cela, un nombre entier de 0 à 3
dans les alcools benzyliques de formule (III).

4. Procédé selon une ou plusieurs des revendications 1 à 3, un carbonate de métal alcalin, en particulier un carbonate de sodium, étant utilisé comme catalyseur dans la première étape du procédé.

5. Procédé selon une ou plusieurs des revendications 1 à 3, le catalyseur étant utilisé dans une quantité de 0,5 à 5, en particulier de 1 à 3% en poids par rapport au carbonate dialkylique de formule (II).

6. Procédé selon une ou plusieurs des revendications 1 à 5, dans lequel le procédé est exécuté dans la première étape à une température de 60 à 160°C, de préférence de 80 à 140°C et à une pression de 0,0001 à 0,1 Mpa.

7. Procédé selon une ou plusieurs des revendications 1 à 6, le carbonate dialkylique de formule (II) étant utilisé avec l'alcool benzylique de formule (III) dans un rapport molaire de (1-8):1, de préférence de (1,5-6):1 et, en particulier, (2-4): 1 dans la première étape du procédé.

8. Procédé selon une ou plusieurs des revendications 1 à 7, le procédé étant exécuté dans la deuxième étape à une température de 60 à 160°C, de préférence de 80 à 140°C et à une pression de 0,01 à 1 Mpa, de préférence 0,1 Mpa.

9. Procédé selon une ou plusieurs des revendications 1 à 8, l'hydrate d'hydrazine étant utilisé par rapport au carbonate dibenzylique dans un rapport molaire de (1-3):1, de préférence (1-2):1 dans la deuxième étape.

10. Procédé selon une ou plusieurs des revendications 1 à 9, le mélange réactif étant traité après la fin de la deuxième étape par distillation, en particulier par entraînement à la vapeur d'eau ou par cristallisation, en particulier cristallisation en solvant ou cristallisation de matière fondue.
